(19) **Europäisches Patentamt**

**European Patent Office**

**Office européen des brevets**

(11) Veröffentlichungsnummer: **0 114 159**
**B1**

# (12) EUROPÄISCHE PATENTSCHRIFT

(45) Veröffentlichungstag der Patentschrift:
08.03.89

(51) Int. Cl.⁴: **C 12 P 7/06**

(21) Anmeldenummer: **84890006.4**

(22) Anmeldetag: **10.01.84**

(54) **Verfahren zur Gewinnung von Äthanol aus vergärbaren Zuckerlösungen.**

(30) Priorität: **13.01.83 AT 106/83**

(43) Veröffentlichungstag der Anmeldung:
**25.07.84 Patentblatt 84/30**

(45) Bekanntmachung des Hinweises auf die Patenterteilung:
**08.03.89 Patentblatt 89/10**

(84) Benannte Vertragsstaaten:
**DE FR SE**

(56) Entgegenhaltungen:
**EP-A-0 044 428**
**DE-A-3 204 910**
**GB-A-1 109 311**
**GB-A-2 021 639**
**GB-A-2 075 053**
**US-A-4 337 123**

**Die Akte enthält technische Angaben, die nach dem Eingang der Anmeldung eingereicht wurden und die nicht in dieser Patentschrift enthalten sind.**

(73) Patentinhaber: **VOEST- ALPINE Aktiengesellschaft, Friedrichstrasse 4, A-1011 Wien (AT)**

(72) Erfinder: **Cvitas, Vilim, Dipl.- Ing., Voltastrasse 29, A-4040 Lins (AT)**
Erfinder: **Faltejsek, Karl, Dipl.- Ing., Lüfteneggerstrasse 6, A-4020 Linz (AT)**
Erfinder: **Hanke, Reinhart, Dipl.- Ing., Annaberggasse 2, A-8700 Leoben (AT)**
Erfinder: **Treso, Bertalan, Kunigundenweg 11a, A-8707 Leoben (AT)**

(74) Vertreter: **Haffner, Thomas M., Dr., Patentanwaltskanzlei Dipl.- Ing. Adolf Kretschmer Dr. Thomas M. Haffner Schottengasse 3a, A-1014 Wien (AT)**

**Beschreibung**

Die Erfindung bezieht sich auf ein Verfahren zur Gewinnung von Äthanol aus vergärbaren Zuckerlösungen. Aus der EP-A-0 044 428 ist es bereits bekannt, zur Herstellung von Alkohol aus Stärke oder stärkehaltigen Rohstoffen, eine Schlempe zu gewinnen, welche durch Zerkleinern, thermischen Aufschluß und Verzuckerung der stärkehaltigen Rohstoffe gebildet wird. Es sind eine Reihe von zuckerhaltigen Rohstoffen bekannt, aus welchen unmittelbar Zuckerlösungen auf extraktivem Weg hergestellt werden können, und es ist bereits bekannt, derartige Zuckerlösungen bis zur Glucose abzubauen und eine auf diese Weise erhaltene Maische zu vergären. Der Gärprozeß verlangt hiebei je nach den eingesetzten Rohstoffen bzw. der eingesetzten Maische unterschiedliche Bedingungen und erfordert einen relativ langen Reaktionszeitraum für den Abschluß der Gärung. Nachteilig bei den bekannten Gärverfahren ist hiebei die Tatsache, daß die Maischen bzw. gärfähigen Substrate, im Verlauf der Gärung unterschiedliche Konzentrationen sowohl an vergärbarem Zucker als auch an Gärungsprodukten aufweisen, welche mit zunehmender Gärung die Reaktionsgeschwindigkeit bzw. die Gärungsgeschwindigkeit stark beeinflussen. Es ist weiter nachteilig, daß mit den bekannten Verfahren immer nur ein mehr oder minder reiner Alkohol und in der Regel mit geringer Konzentration hergestellt werden kann.

Die üblicherweise aus stärkehaltigen Rohstoffen durch Verzuckerung vergärbaren Zuckerlösungen weisen relativ geringe Zuckerkonzentrationen auf. Mit Rücksicht auf eine raschere Gärung ist es in diesen Fällen vorteilhaft, eine Volumensreduktion bei gleichzeitiger Erhöhung der Zuckerkonzentration vorzunehmen.

Die Erfindung zielt nun darauf ab, eine besonders einfache Verfahrensweise zu schaffen, mit welcher ein unmittelbar vergärbares Substrat mit einer für die nachfolgende Gärung günstigen Zuckerkonzentration geschaffen wird, und auf die apperativ und ernergetisch aufwendigen Eindickungsgeräte verzichtet werden kann. Zur Lösung dieser Aufgabe besteht die Erfindung im wesentlichen darin, daß der gebildete Hefeschlamm von der entstandenen Mischung abgetrennt wird, daß der Hefeschlamm in den Reaktionsraum eingebracht wird, daß die Hefe nach Abschluß des Gärungsprozesses aus dem Gärbehälter ausgetragen wird und nach einer Belüftung und nach Versetzen mit einer Nährstofflösung, insbesondere mit Kohlenstoffträgern und/oder Nährsalzen, zumindest teilweise der Zuckerlösung vor dem Eintragen in den Gärbehälter zur Herstellung der Mischung rückgeführt wird. Durch den Zusatz von Hefe wird nach einer Reaktionszeit von etwa 10 Minuten Glucose aus der Zuckerlösung von der Hefe aufgenommen. Der Einsatz des Hefeschlammes, mit welchem die Glucose angereichert ist, führt zu einer besonders raschen Vergärung des in der Zuckerlösung enthaltenen Zuckers und auf Grund der schonenden Verfahrensbedingungen kann nicht nur der Großteil der handelsüblichen Hefen bei guten Gärleistungen verwendet werden, sondern es ist auch ein Führen der Hefe im Kreislauf und damit eine besonders umweltschonende Verfahrensführung möglich. Zu diesem Zweck wird erfindungsgemäß die Hefe nach Abschluß des Gärprozesses aus dem Gärbehälter ausgetragen und nach einer Belüftung und nach Versetzen mit einer Nährstofflösung, insbesondere mit Kohlenstoffträgern und/oder Nährsalzen, zumindest teilweise der Zuckerlösung vor dem Eintragen in den Gärbehälter zur Herstellung der Mischung rückgeführt. Die überschüssige Hefe kann abgetrennt werden und einer anderen Verwendung zugeführt werden.

Die Hefe wird in vorteilhafter Weise, insbesondere dann wenn der gebildete Alkohol kontinuierlich abgeführt, beispielsweise bereits während der Gärung destillativ abgetrennt wird, nie oder nur kurzzeitig höheren Konzentrationen an Gärungsprodukten ausgesetzt, so daß eine bessere Vitalität des biologischen Materials vorliegt. Es ist im Rahmen des erfindungsgemäßen Verfahrens eine Regeneration der Hefe nur in geringem Umfang notwendig.

Die Abtrennung des Hefeschlammes aus der Mischung mit der Zuckerlösung kann in besonders vorteilhafter Weise durch Flotation erfolgen, wobei die bereits einsetzende Gasentwicklung ausgenutzt werden kann und eine Flockung durch Zusatz von Polyelektrolyten vorgesehen sein kann. Flockung, Flotation und Hefeabtrennung können bei Gärungstemperatur (30 bis 35° C) erfolgen. Gegebenenfalls wird bereits die Mischung der Hefe mit der Glucoselösung bei Gärungstemperatur durchgeführt. Die Flockung kann mit Vorteil so durchgeführt werden, daß die vom Hefeschlamm vor dem Einbringen desselben in den Gärbehälter abgetrennte Lösung zusammen mit Polyelektrolyten der Flockung zugesetzt wird, wobei auf diese Weise die noch zuckerhaltige Lösung, welche vom Hefeschlamm abgetrennt wurde, zumindest teilweise im Kreislauf geführt werden kann. Eine Führung der Hefe im Kreislauf wird insbesondere durch die kurze Gärungsdauer ermöglicht. Zur Regeneration der Hefe wird diese nach dem Ausbringen aus dem Gärbehälter einer aeroben Phase unterworfen, wobei in regelmäßigen Abständen Hefe abgezogen werden kann und für die Futtermittelproduktion verwendet werden kann.

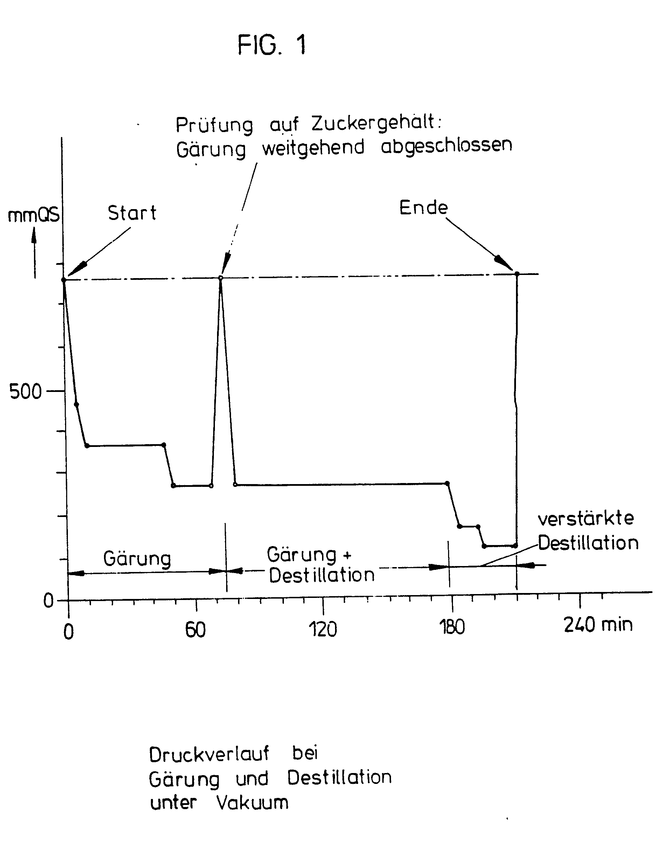

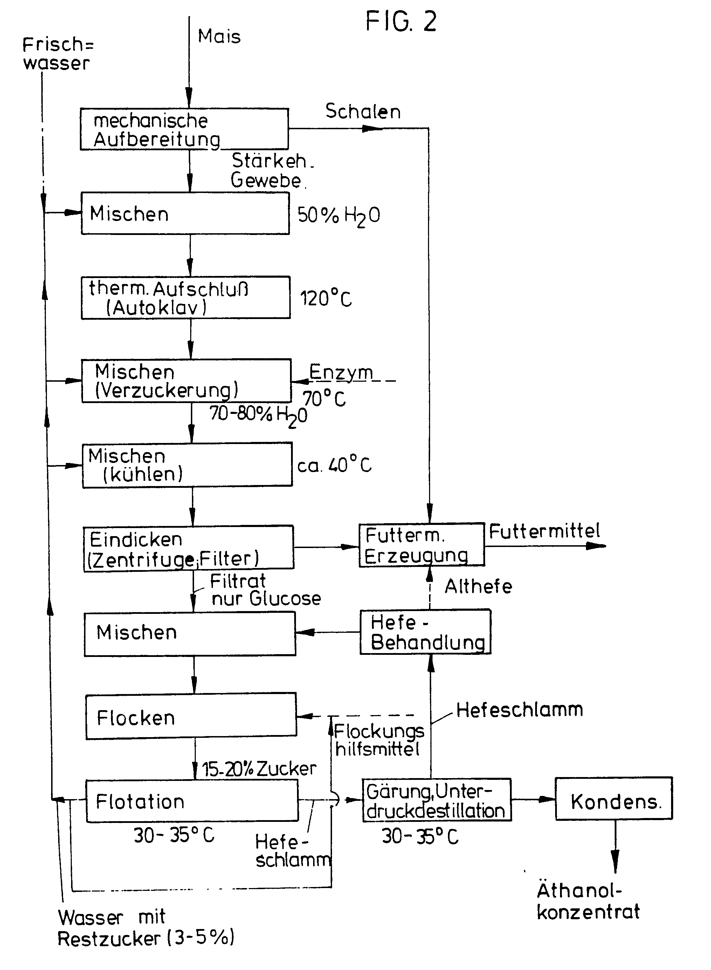

Die Erfindung wird nachfolgend an Hand der in der Zeichnung dargestellten Druckverlaufkurve und eines Ausführungsbeispieles näher erläutert. In der Zeichnung zeigt Fig. 1 eine Druckverlaufkurve für das erfindungsgemäße Gärverfahren und Fig. 2 ein vereinfachtes Fließschema für eine vollständige Aufbereitung von stärkehaltigen Rohstoffen zu Alkohol.

Die angegebenen Mengen im Ausführungsbeispiel beziehen sich hiebei auf 1 l Zuckerlösung.

| | |
|---|---|
| Glucoseeinsatz = | 182,8 g |
| Hefeeinsatz = | 1,47 kg Schlamm mit ca. 4 % TS |
| Reaktionszeit = | 10 Minuten |
| Polyelektrolyteinsatz = | 320 mg ($\hat{=}$ 1,75g/kg Glucose) |
| Reaktionszeit = | 10 Minuten |
| Flotationszeit = | 7,0 Minuten |
| abgezogene Zuckerlösung = | 1,10 l |
| abgezogener Restzucker = | 52,9 g Glucose |
| Zucker mit Hefeschlamm zur Gärung = | 129,9 g. |

Die Gärung wurde analog der Darstellung der Zeichnungsfigur so durchgeführt, daß der Druck unmittelbar nach dem Start auf 350 mbar abgesenkt wurde. Nach etwa 45 Minuten wurde der Druck weiter auf 260 mbar abgesenkt und nach etwa 70 Minuten der Zuckergehalt bestimmt. Innerhalb dieser Zeit ist die Gärung bereits weitgehend abgeschlossen und es tritt bereits während dieser Zeit eine Destillation des entstehenden Alkohols ein. Nach etwa 180 Minuten wurde der Druck im Reaktionsraum weiter stufenweise abgesenkt, um eine vollständige Destillation zu erzielen und die Äthanolausbeute zu vervollständigen. Je 1 l Zuckerlösung wurden hiebei eine Menge von 0,34 l Destillat mit einem Äthanolgehalt von 0,077 l, das heißt, 60,7 g Äthanol, gewonnen. Die Ausbeute des adsorbierten Zuckers beträgt hiebei

$$\frac{60,7}{129,9 \cdot 0,511} = 0,915$$

Glucose konnte im Hefeschlamm nicht nachgewiesen werden.

Für die Durchführung des gesamten Verfahrens ergibt sich folgender Zeitablauf:

| | |
|---|---|
| Glucoseadsorption | 0,17 h |
| Flockung | 0,02 h |
| Flotation | 0,12 h |
| Gärung + Destillation | 3,50 h |
| gesamte Prozeßdauer = | 3,81 h |

Die im Kreislauf geführte Lösung, welche vom Hefeschlamm vor dem Einbringen in den Reaktionsraum abgetrennt wurde, enthält durchschnittlich 3 bis 5 Gew.-% Glucose.

Der Verfahrensablauf einer Aufbereitung von zuckerhaltigen Rohstoffen bis zum Äthanol ist schematisch in Fig. 2 dargestellt. Aus diesem Fließschema ergeben sich auch die erfindungsgemäß vorgeschlagenen Kreislaufführungen zum Zwecke der Verbesserung der Energieausbeute bzw. der Glucoseausbeute.

## Patentansprüche

1. Verfahren zur Gewinnung von Äthanol aus vergärbaren Zuckerlösungen, bei welchem die Zuckerlösung vor dem Einbringen in einen Gärbehälter mit Hefe versetzt wird, dadurch gekennzeichnet, daß der gebildete Hefeschlamm von der entstandenen Mischung abgetrennt wird, daß der Hefeschlamm in den Reaktionsraum eingebracht wird, daß die Hefe nach Abschluß des Gärprozesses aus dem Gärbehälter ausgetragen wird und nach einer Belüftung und nach Versetzen mit einer Nährstofflösung, insbesondere mit Kohlenstoffträgern und/oder Nährsalzen, zumindest teilweise der Zuckerlösung vor dem Eintragen in den Gärbehälter zur Herstellung der Mischung rückgeführt wird.

2. Verfahren nach Anspruch 1, dadurch gekennzeichnet, daß die Mischung vor dem Abtrennen des Hefeschlammes, beispielsweise durch Flotation, einer Flockung unterworfen wird.

3. Verfahren nach Anspruch 2, dadurch gekennzeichnet, daß die vom Hefeschlamm vor dem Einbringen desselben in den Gärbehälter abgetrennte Lösung zusammen mit Polyelektrolyten der Flockung zugesetzt wird.

## Claims

1. Process for the preparation of ethanol from fermentable sugar solutions, wherein yeast is added to the sugar solution prior to its feeding into a fermenting vat, characterised in that the formed yeast sludge is separated from the produced mixture, that the yeast sludge is fed into the reaction chamber, that after termination of the fermentation process the yeast is discharged from the fermenting vat and, after aeration and mixing with a nutritive solution, in particular with carbon carriers and/or nutrient salts, is at least partially returned to the sugar solution prior to feeding said solution into the fermenting vat for preparing the mixture.

2. Process as claimed in claim 1, characterised in that prior to the separation of the yeast sludge, e.g. by flotation, the mixture is subjected to flocculation.

3. Process as claimed in claim 2, characterised in that the solution separated from the yeast sludge prior to its feeding into the fermenting vat is added to the flocculation together with polyelectrolytes.

## Revendications

1. Procédé d'obtention d'éthanol à partir de solutions de sucre fermentescibles, dans lequel la solution de sucre est additionnée de levure avant d'être introduite dans une cuve de fermentation, caractérisé en ce que la boue de levure formée est séparée du mélange obtenu, en ce que la boue de levure est introduite dans l'enceinte de réaction, en ce que la levure est extraite de la cuve de fermentation après achèvement du processus de fermentation, et est renvoyée, au moins en partie, vers la solution de sucre pour que soit réalisé le mélange, après aérage et après addition d'une solution nutritive, en particulier de supporte de carbone et/ou de sels nutritifs, avant l'introduction dans la cuve de fermentation.

2. Procédé selon la revendication 1, caractérisé en ce que le mélange est soumis à une floculation, par exemple par flottation, avant séparation de la boue de levure.

3. Procédé selon la revendication 2, caractérisé en ce que la solution séparée de la boue de levure, avant introduction de celle-ci dans la cuve de fermentation, est ajoutée à la floculation, en même temps que des polyélectrolytes.

# FIG. 1

Druckverlauf bei
Gärung und Destillation
unter Vakuum

# FIG. 2